# EUROPÄISCHE PATENTANMELDUNG

(11) **EP 1 484 050 A2**
(43) Veröffentlichungstag der Anmeldung: **08.12.2004**
(21) Anmeldenummer: 04012403.4
(22) Anmeldetag: 26.05.2004
(51) Int. Cl.: A61K 7/42, A23L 1/305

(54) **Zubereitung zur Herstellung von Pflege- und kosmetischen Produkten sowie für Nahrungsergänzungsmittel**

(30) Priorität: 02.06.2003 DE 20308638 U; 22.08.2003 DE 20313122 U
(71) Anmelder: STS TENSID SERVICE GmbH, 68649 Gross-Rohrheim (DE)
(72) Erfinder: Schnittker, Heinz-Peter, 68649 Gross-Rohrheim (DE)
(74) Vertreter: Richter, Werdermann, Gerbaulet & Hofmann

(57) **Zusammenfassung**

Die Zubereitung zur Herstellung von Pflege- und kosmetischen Produkten sowie von Nahrungsergänzungsmittel-Produkten besteht aus einem geschmacksneutralen Pulver der Süßwasserperle, insbesondere einer chinesischen Süßwasserperle oder einem geschmacklich artspezifischen Pulver eines Süßwasserperlen-Hydrolysats mit einer Korngrößenverteilung von 0,2 µm bis 600 µm mit einer spezifischen Oberfläche von 1,0 m²/g bis 1,5 m²/g und einem Gehalt an organischen Polymeren von ≥ 1,85 %.

## Beschreibung

Die Erfindung betrifft eine Zubereitung zur Herstellung von Pflege- und kosmetischen Produkten sowie für Nahrungsergänzungsmittel,

Die Kosmetik dient sowohl der äußerlichen Verschönerung, als auch der Pflege der Haut und der Haare. Heutige kosmetische Mittel sollen darüber hinaus für die Gesundheit unbedenklich und die Umwelt nicht belastend sein.

Kosmetische Mittel sind dazu bestimmt, die Haut, Haare, Nägel und Lippen usw. zu reinigen, sie zu schützen, oder ihr Aussehen zu verändern.

So ist die Haut neben dem Haar der wichtigste Träger für kosmetische Mittel. Sie übt als größtes Sinnesorgan des Menschen zahlreiche lebenswichtige Funktionen aus. Neben ihrer Schutzfunktion gegenüber Umwelteinflüssen ist die Haut an verschiedenen Stoffwechsel-, Speicherungsund Regulationsvorgängen des Körpers beteiligt. Sie leistet sowohl Schutz vor mechanischen Einwirkungen als auch vor UV-Strahlen. Sie dient zudem der Immunabwehr, der Wärmeregulation und leistet Wahrnehmungsfunktionen. Somit sind kosmetische Mittel für die Haut zur Reinigung, Desodorierung, zum Schutz gegen UV-Strahlen oder als Make-up erforderlich. Weitere Wirkungsweisen wie Feuchtigkeitsregulation, Hautquellung, Antifaltenwirkung, Hautverträglichkeitssteigerung oder Effizienz gegenüber Hautunreinheiten werden meistens auch angestrebt. Außerdem sollen die Mittel ein belebendes Gefühl und jugendliches Aussehen bewirken.

Neben der Pflege der Haut ist auch das Haar zu pflegen und zu erhalten. Die Reinigung der Haare und der Kopfhaut von körpereigenem Fett, Hautabschilferungen, Schmutz und Gerüchen aus der Umwelt und gegebenenfalls von Rückständen anderer Haarbehandlungsmittel sind wichtig. Zu den kosmetischen Mitteln zur Haarbehandlung gehören neben den Shampoos auch Haarpflegemittel. Das stetig nachwachsende Haar ist nur für eine zeitlich begrenzte Haltbarkeit vorgesehen. Dies zeigt sich deutlich beim Vergleich des frisch nachgewachsenen Haares mit den bereits mehrere Monate oder Jahre alten Haarspitzen. Während der in der Nähe der Kopfhaut befindliche Teil des Haares eine völlig intakte, nahezu geschlossene Schuppenschicht zeigt, weist das gleiche Haar mit zunehmendem Abstand von der Kopfhaut eine Schwächung, in extremen Fällen sogar den völligen Verlust der Schuppenschicht auf. Wesentliche Ursachen für diesen Qualitätsverlust des Haares sind der Einfluss des Sonnenlichtes, mechanische Beanspruchungen durch intensives Kämmen oder Bürsten und unvermeidliche Nebenreaktionen bei chemischen Prozessen wie Dauerwellen und Haarfärbungen, insbesondere Blondierungen. Ein Ziel der Haarpflege ist es deshalb, den Naturzustand des frisch nachgewachsenen Haares über einen möglichst langen Zeitraum zu erhalten und im Falle seines Verlustes wieder herzustellen. Seidiger Glanz, geringe Porosität und ein angenehmes, glattes Gefühl gelten als Merkmale für natürliches und gesundes Haar. Als Haarpflegemittel werden beispielsweise Vorbehandlungsmittel, Haarwässer, Haarspülungen und Haarkuren verwendet.

Bekannt sind eine Vielzahl von Zubereitungen kosmetischer Produkte auf der Basis von Pflanzen bzw. Pflanzenextrakten (z. B. DE 101 21 092 A1).

Bekannt ist ferner eine Wirkstoffkombination aus Perlmutt und Ascorbinsäure in kosmetischen Zubereitungen wie Cremes, Ölen, Wässer, Puder und Pulver (DE 299 10 271 U1).

Die DE 296 14 226 U1 beschreibt ein Dauerwellmittel und Fixiermittel für Dauerwellen, bei dem dem Mittel ein Perlenproteinhydrolysat zugesetzt wird, um dem Haar nach dem Dauerwellen Glanz und eine gute Kämmbarkeit zu verleihen.

Eine kosmetische Zusammensetzung zu dekorativen Zwecken beschreibt die DE 35 30 902 A1, die ein flüssiges, hochviskoses oder festes, hautverträgliches Trägermaterial und reines Pulver von Edelsteinen oder Edeisteinmischungen umfasst, wobei auch Korallen und Perlen eingesetzt werden können. Die Zusammensetzung wird verwendet zur Herstellung von Pulver-Lidschatten, Puderlidschatten, Körperpuder und von Lidschattencreme, wobei durch die eingesetzten Edelsteine und Edelsteinmischungen eine Kosmetik erhalten werden soll, die sich durch Farbreinheit, Glanzfähigkeit und Brillanz, auszeichnet und somit ausschließlich zu dekorativen Zwecken dient.

Aufgabe der vorliegenden Erfindung ist es, eine Zubereitung der eingangs beschriebenen Art unter Verwendung von Süßwasserperlen, insbesondere chinesische Süßwasserperlen, zu schaffen, bei der die in den Süßwasserperlen enthaltenen Aminosäuren vollständig und unverändert erhalten bleiben, so dass die Zubereitung einsetzbar ist in der Kosmetik mit oder ohne Lichtschutzfaktor und für die menschliche und tierische Ernährung.

Gelöst wird diese Aufgabe durch Zubereitungen mit den Merkmalen der Ansprüche 1 bis 4.

Die erste erfindungsgemäße Zubereitung zur Herstellung von Pflege- und kosmetischen Produkten sowie von Nahrungsergänzungsmittel-Produkten nach Anspruch 1 umfasst ein geschmacksneutrales Pulver der Süßwasserperle, insbesondere einer chinesischen Süßwasserperle, mit einer Korngrößenverteilung von 0,2 µm bis 600 µm mit einer spezifischen Oberfläche von 1,0 m²/g bis 1,5 m²/g und einem Gehalt an organischen Polymeren (Protein, Chitin) von ≥ 1,85 %.

Die zweite erfindungsgemäße Zubereitung nach Anspruch 2 umfasst ein geschmacksneutrales Pulver der Süßwasserperle, insbesondere einer chinesischen Süßwasserperle, mit einer Korngrößenverteilung von 0,3 µm bis 200 µm mit einer spezifischen Oberfläche von 1,0 m²/g bis 1,5 m²/g und einem Aminosäuregehalt von ≥ 2 %.

Eine dritte erfindungsgemäße Zubereitung nach Anspruch 3 umfasst ein geschmacklich artspezifisches Pulver eines Süßwasserperlen-Hydrolysats, insbesondere eines chinesischen Süßwasserperlen-Hydrolysats, mit einer Korngrößenverteilung von 0,2 µm bis 200 µm mit einer spezifischen Oberfläche von 1,0 m²/g bis 1,5 m²/g und einem Gehalt an organischen Polymeren (Protein, Chitin) von ≥ 0,55 %.

Eine vierte erfindungsgemäße Zubereitung nach Anspruch 4 umfasst ein geschmacklich artspezifisches Pulver eines Süßwasserperlen-Hydrolysats, insbesondere eines chinesischen Süßwasserperlen-Hydrolysats, mit einer Korngrößenverteilung von 0,5 µm bis 250 µm mit einer spezifischen Oberfläche von 1,0 m²/g bis 1,5 m²/g und einem Aminosäuregehalt von ≥ 2 %.

Überraschenderweise wurde aufgefunden, dass pulverisierte chinesische Süßwasserperlen auch in Form eines Hydrolysats mit der erfindungsgemäßen Korngrößenverteilung beim Hydrolysat und beim reinen Perlenpulver einen hohen Anteil an Aminosäuren aufweisen und dass die Aminosäuren weder zerstört noch in irgendeiner Weise verändert umgewandelt werden, so dass der volle Aminosäurengehalt erhalten bleibt und eingesetzt werden kann.

Das Süßwasserperlen-Pulver enthält neben organischen Polymeren

| | |
|---|---|
| Calcium (Ca) | > 25 % |
| Blei (Pb) | < 0,5 mg/KG |
| Arsen (As) | < 0,5 mg/KG |
| Quecksilber (Hg) | < 0,1 mg/KG |
| Wasser (H²O) | ≤ 2,0 % |

und das Süßwasserperlenpulver-Hydrolysat neben organischen Polymeren

| | |
|---|---|
| Calcium (Ca) | < 20 % |
| Blei (Pb) | < 0,5 mg/KG |
| Arsen (As) | < 0,5 mg/KG |
| Quecksilber (Hg) | < 0,1 mg/KG |
| Wasser (H²O) | ≤ 10 %. |

Die Zubereitungen enthalten folgende Aminosäuren:

Aspartic Acid, Threonine, Serine, Glutamic, Proline, Glycine, Cysteine, Alanine, Valine, Methione, Isoleucine, Leucine, Tyrosine, Phenylalanine, Lysine, Histidine, Arginine, Ornithine.

Außerdem enthalten die Zubereitungen einen synergistischen UV-Schutz, insbesondere einen synergistischen UV-A-Schutz und/oder einen synergistischen UV-B-Schutz. Dabei wird der synergistische UV-B-Schutz in Verbindung mit einer 4-Aminobenzoesäure, Homomenthylsalicylat, 2-Phenylbenzimidazol-5-Sulfonsäure, 4-Dimethylaminobenzoesäure-2-ethylhexylester, 4-Methoxy-zimtsäureisoamylester, 4-Methoxy-zimtsäure-2-ethylhexylester, 3-(4'-Methyl)benzyliden-bornan-2-on, oder anorganischen UV Filtern (Titandioxid bzw. Zinkoxid) und der synergistische UV-A-Schutz in Verbindung mit 2-Hydroxy-4-methoxybenzophenon, 1-(4'-Isopropylphenyl)3-phenyl-1-propan-1,3-dion oder 1-(4-t-Butylphenyl)-3-(4-methoxyphenyl)-propan-1,3-dion oder anorganischen UV-Filtern (Titandioxid bzw. Zinkoxid) erzielt.

Des weiteren umfasst die Erfindung die Verwendung eines geschmacksneutralen Pulvers mit einer Korngrößenverteilung von 0,2 µm bis 600 µm mit einer spezifischen Oberfläche von 1,0 m²/g bis 1,5 m²/g und einem Gehalt an organischen Polymeren/Aminosäuren (Protein, Chitin) von ≥ 1,85 % der Süßwasserperle, insbesondere einer chinesischen Süßwasserperie, ais Zubereitung für die Herstellung von Pfiege- und kosmetischen Produkten sowie für Nahrungsergänzungsmittel-Produkten oder als Zubereitung für die Herstellung von Lichtschutzmitteln.

Die Erfindung umfasst ferner die Verwendung eines geschmacklich artspezifischen Pulvers mit einer Korngrößenverteilung von 0,2 µm bis 200 µm mit einer spezifischen Oberfläche von 1,0 m²/g bis 1,5 m²/g und einem Gehalt an organischen Poylmeren/Aminosäuren (Protein, Chitin) von ≥ 0,55 % eines Süßwasserperlen-Hydrolysats, insbesondere eines chinesischen Süßwasserperlen-Hydrolysats, als Zubereitung für die Herstellung von Pflege- und kosmetischen Produkten sowie für Nahrungsergänzungsmittel-Produkten oder als Zubereitung für die Herstellung von Lichtschutzmitteln.

Besonders vorteilhaft lässt sich die Zubereitung als Sonnenschutzmittel in Form von Cremes oder einer anderen geeigneten, auf die menschliche Haut auftragbaren Form, einsetzen.

Die Zubereitung bzw. das Lichtschutzmittel kann mindestens einen UV-Filter umfassen, wobei der UV-Filter ein UV-B-Filter und/oder ein UV-A-Filter ist. Des weiteren kann die Zubereitung bzw. das Lichtschutzmittel ein Mikropigment umfassen, wobei das Mikropigment ultrafeines Titandioxid ist.

Die Herstellung der Zubereitung erfolgt dabei durch
- Auswahl von geeigneten Süßwasserperlen
- Waschen und Desinfizieren der Perlen
- Trocknen der Perlen
- Zerkleinern der Süßwasserperlen zu einem Pulver mittels eines Ultra-Mikro-Mahlprozesses
- Saure Hydroiyse für die Herstellung des Süßwasserperlen-Hydrolysats
- Trocknung des Hydrolysats
- Verpackung des Endproduktes.

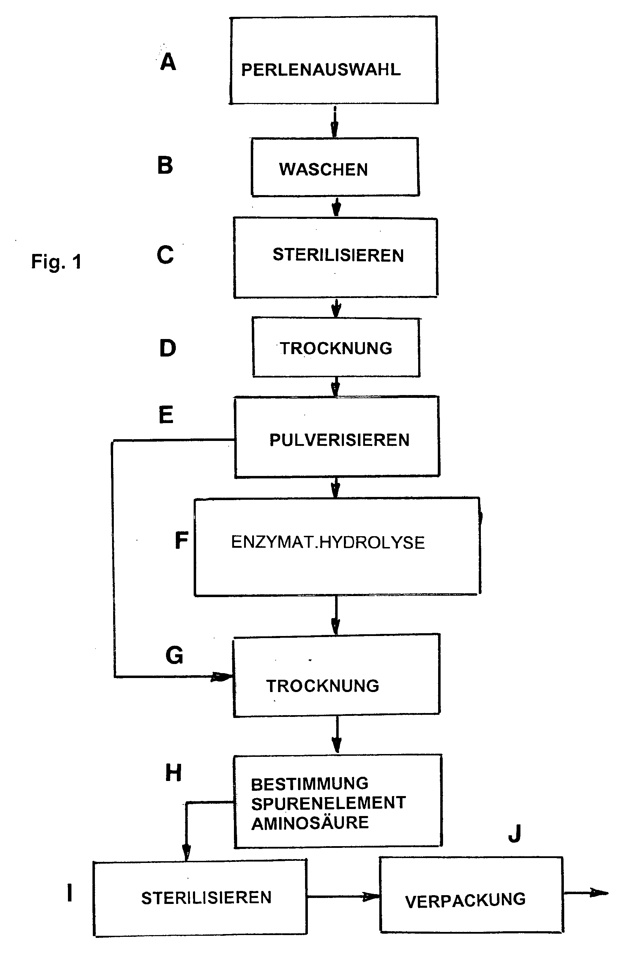

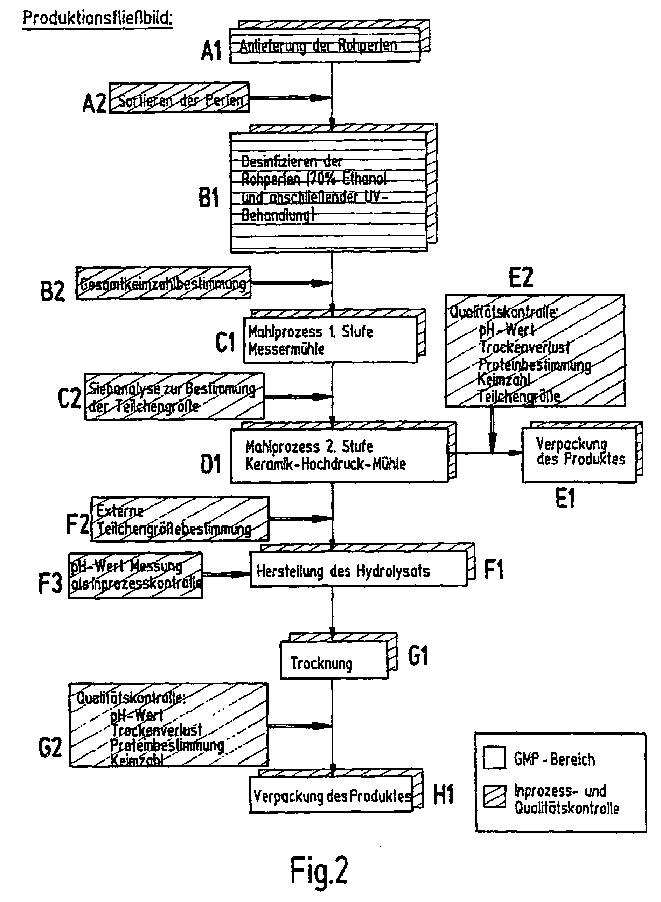

Das Verfahren zur Herstellung der erfindungsgemäßen Zubereitungen wird anhand der Fig. 1 und Fig. 2 erläutert:

Nach Fig. 1 erfolgt zunächst in Stufe A eine Auswahl der für die Herstellung der Zubereitung geeigneten Süßwasserperlen. In den Stufen B und C erfolgt das Waschen und Sterilisieren der zu verarbeitenden Süßwasserperlen, wobei die Stufen B und C auch zusammengelegt werden können. Danach schließt sich in Stufe D eine Trocknung der Süßwasserperlen an. Die so vorbereiteten Süßwasserperlen werden zu einem Pulver mit einer Korngrößenverteilung von 0,3 µm bis 200 µm bzw. 0,5 µm bis 250 µm und mit einer spezifischen Oberfläche von 1,0 m²/g bis 1,5 m²/g vermittels eines Ultra-Mikro-Mahlprozesses in Stufe E zerkleinert. Bei der Herstellung des Süßwasserperlen-Hydrolysats erfolgt dann in Stufe F die enzymatische Hydrolyse. In Stufe G erfolgt dann eine Trocknung. Es folgt dann in Stufe H eine Bestimmung der Spurenelemente und der Aminosäuren. Stufe I sieht eine Sterilisation des Pulvers vor. In Stufe J erfolgt die Verpackung in Dosen, Beutel u. dgl.

Nach dem Produktionsfließbild gemäß Fig. 2 erfolgt nach der Anlieferung in Stufe A1 der Rohperlen ein Sortieren A2 der Perlen in Stufe A2. Hierauf wird in Stufe B1 die Desinfizierung der Rohperlen durch Behandlung in 70 %igem Ethanol und eine anschließende Behandlung mit UV-Licht vorgenommen. Nach einer Gesamtkeimzahlbestimmung in Stufe B2 werden die Perlen einem ersten Mahlprozess vermittels einer Messermühle in Stufe C1 unterworfen. Vor dem Einleiten eines zweiten Mahlprozesses vermittels einer Keramik-Hochdruckmühle in der Stufe D1 wird in einer vorgeschalteten Stufe C2 eine Siebanalyse zur Bestimmung der Teilchengröße durchgeführt. Das Produkt des zweiten Mahlprozesses wird dann in der Stufe E verpackt. Der Verpackung ging in Stufe E2 eine Qualitätskonirolle voraus, die eine Messung des pH-Wertes und des Trockenverlustes, eine Proteinbestimmung, eine Bestimmung der Keimzahl und der Teilchengröße umfasst.

Aus dem Produkt des zweiten Mahlprozesses gemäß Stufe D1 kann dann des weiteren das Hydrolysat gemäß Stufe F1 hergestellt werden, dem in Stufe F2 eine externe Teilchengrößenbestimmung vorangegangen ist. Außerdem erfolgt in Stufe F3 eine pH-Wertmessung als Inprozesskontrolle. An die Stufe F1 schließt sich dann in Stufe G1 ein Trocknungsprozess an und nach einer Qualitätskontrolle in Stufe G2 mit pH-Wertmessung, Bestimmung des Trockenverlustes, der Proteinbestimmung und der Bestimmung der Keimzahl erfolgt dann in Stufe H1 die Verpackung des Endproduktes, des Perlpulver-Hydrolysats.

Das Produktionsfließbild gemäß Fig. 2 zeigt die Herstellung der erfindungsgemäßen Zubereitung in Form des Pulvers der Süßwasserperle und des Pulvers des Süßwasserperlen-Hydrolysats in einem mehrstufigen Verfahren.

Die Erfindung sieht ferner die Verwendung der erfindungsgemäßen Zubereitung für die Herstellung von Pflege- und kosmetischen Produkten sowie für Nahrungsergänzungsmittel-Produkten unter Aufrechterhaltung des vollen Gehaltes an Aminosäuren vor.

Die erfindungsgemäße Zubereitung enthält gemahlene chinesische Süßwasserperlen und/oder deren Hydrolysat und kann außerdem auch noch die üblichen Hilfs- und Trägerstoffe enthalten.

Auch weitere Wirkstoffe können hinzugefügt werden. Die Zubereitungen können auf Wasser oder Öl basieren als Trägerstoffe oder sind wasserfrei. Auch in Form von Emulsionen können die Zubereitungen, insbesondere zum Schutz von thermolabilen Wirkstoffen, wie Aminosäuren, eingesetzt werden.

Die erfindungsgemäß gemahlenen chinesischen Süßwasserperlen können des weiteren in kosmetischen Zubereitungen zur Herstellung von Cremes, Lotionen, wasser- und abriebstabilen Kosmetika, liposomen-haltigen Pflegeprodukten, Sonnenschutzmitteln und Mitteln gegen die Hautalterung eingesetzt werden.

Der Hauptwirkstoff der erfindungsgemäßen Zubereitungen ist ein Pulver von gemahlenen chinesischen Süßwasserperlen und/oder deren Hydrolysat.

Die kosmetischen Zubereitungen können unter Verwendung des Süßwasserperlenpulvers auf wässriger oder ölhaltiger Basis hergestellt werden; so in Form von Pudern, Gesichtsmasken, Make-ups, Lotionen, Seifen, Cremes, Haarwässern, Badesalzen, Badezusätzen, Duschgelen, Lippenstiften oder Ölen, oder aber auf wasserfreier Basis in Form von Pudern, Seifen, Gesichtsmasken, Make-ups, Haarwässern, Lippenstiften oder Lotionen.

Der Einsatz von gemahlenen chinesischen Süßwasserperlen in Kosmetika bewirkt aufgrund des hohen Gehaltes an Aminosäuren eine Verbesserung der Hautfeuchtestabilität, eine Antiaging-Wirkung, insbesondere eine Verminderung der Faltenbildung der Haut, eine Glättung der Haut, eine Erhöhung der Hautelastizität sowie einen UV-Schutz.

Die erfindungsgemäßen Zubereitungen sind überraschenderweise auch als Sonnenschutzmittel, beispielsweise in Form von Cremes oder in einer anderen geeigneten, auf die menschliche Haut auftragbaren Form bzw. zur Herstellung von kosmetischen Produkten mit hohem Lichtschutzfaktor einsetzbar. Derartige kosmetische Produkte werden im folgenden als Lichtschutzmittel bezeichnet. Lichtschutzmittel sollen einerseits die Haut vor einem Sonnenbrand schützen und andererseits eine schnelle und tiefe Hautbräunung ermöglichen.

Die Lichtschutzmittel können neben der erfindungsgemäßen Zubereitung weitere, anorganische oder organische Komponenten enthalten, die üblicherweise in Lichtschutzmitteln verwendet werden. Derartige Komponenten umfassen UV-Filter, Pigmente, Mikropigmente und Antioxidantien, ohne auf diese beschränkt zu sein.

UV-Filter umfassen beispielsweise 4-Aminobenzoesäure, Homomenthylsalicylat, 2-Hydroxy-4-methoxybenzophenon; 2-Phenylbenzimidazol-5-sulfonsäure; 4-Dimethylaminobenzoesäure-2-ethylhexylester; 4-Methoxy-zimtsäure-isoamylester; 4-Methoxy-zimtsäure-2-ethylhexylester; 3-(4'-Methyl)benzyliden-bornan-2-on; 1-(4'-Isopropylphenyl)-3-phenyl-1-propan-1,3-dion und 1-(4-t-Butylphenyl)-3-(4-methoxyphenyl)-propan-1,3-dion.

Pigmente und Mikropigmente umfassen beispielsweise Titanoxid und Zinkoxid. Bevorzugt wird die Verwendung von ultrafeinem Titandioxid. Besonders bevorzugt wird ultrafeines Titandioxid mit einem Primärteilchendurchmesser von 10 bis 60 nm.

Antioxidantien umfassen alle antioxidativ wirksamen Substanzen wie beispielsweise Radikalfänger, reduzierende Stoffe, Enzyme oder Chelatbildner. Beispiele derartiger Substanzen sind Superoxid-Dismutase, Tocopherole und Ascorbinsäure.

Kosmetische Zusammensetzungen, die unter Verwendung der erfindungsgemäßen Zubereitungen, hergestellt worden sind, besitzen einen hohen Lichtschutzfaktor, insbesondere einen hohen UV-Schutz, und weisen synergetische Effekte auf.

Eine bevorzugte kosmetische Zusammensetzung zur Verwendung als Lichtschutzmittel umfaßt die erfindungsgemäße Zubereitung, ultrafeines Titandioxid sowie einen oder mehrere der obengenannten UV-Filter.

Der oder die UF-Filter werden in Abhängigkeit von dem beabsichtigten Absorptionsbereich des Lichtschutzmittels ausgewählt. Unter UV-B-Filtern werden UV-Filter verstanden, die den Teil des Spektrums der elektromagnetischen Strahlung mit einer Wellenlänge von 280 bis 314 nm absorbieren. Beispiele für UV-B-Filter sind 4-Aminobenzoesäure, Homomenthylsalicylat, 2-Phenylbenzimidazol-5-sulfonsäure, 4-Dimethylaminobenzoesäure-2-ethylhexylester, 4-Methoxy-zimtsäure-isoamylester, 4-Methoxy-zimtsäure-2-ethylhexylester und 3-(4'-Methyl)benzylidenbornan-2-on. Unter UV-A-Filtern werden UV-Filter verstanden, die den Teil des Spektrums der elektromagnetischen Strahlung mit einer Wellenlänge 315 bis 400 nm absorbieren. Beispiele für UV-A-Filter sind 2-Hydroxy-4-methoxybenzophenon, 1-(4'-Isopropylphenyl)-3-phenyl-1 -propan-1,3-dion und 1-(4-t-Butylphenyl)-3-(4-methoxyphenyl)-propan-1,3-dion. Werden Kombinationen aus UV-A-Filtern und UV-B-Filtern verwendet, werden Lichtschutzmittel mit Breitbandfiltereigeschaften erhalten.

Die Lichtschutzmittel können in Form von Sonnenöl, Sonnenmilch und Sonnencremes zubereitet werden. Das Lichtschutzmittel kann darüber hinaus als Shampoo zubereitet werden. Das Shampoo dient dann zum Schutz der Haare.

Die unter Verwendung der erfindungsgemäßen Zubereitung hergestellten Lichtschutzmittel förden die Hautpflege und tragen zur Gesunderhaltung von Haut und Haaren bei. Dabei wird die Hautstruktur verbessert.

Die voranstehend gemachten Angaben beziehen sich auf Gew.-%.

## Patentansprüche

1. Zubereitung zur Herstellung von Pflege- und kosmetischen Produkten sowie von Nahrungsergänzungsmittel-Produkten,
**gekennzeichnet durch** ein geschmacksneutrales Pulver der Süßwasserperle, insbesondere einer chinesischen Süßwasserperle, mit einer Korngrößenverteilung von 0,2 µm bis 600 µm mit einer spezifischen Oberfläche von 1,0 m²/g bis 1,5 m²/g und einem Gehalt an organischen Polymeren (Protein, Chitin) von ≥ 1,85 %.

2. Zubereitung zur Herstellung von Pflege- und kosmetischen Produkten sowie von Nahrungsergänzungsmittel-Produkten,
**gekennzeichnet durch** ein geschmacksneutrales Pulver der Süßwasserperle, insbesondere einer chinesischen Süßwasserperle, mit einer Korngrößenverteilung von 0,3 µm bis 200 µm mit einer spezifischen Oberfläche von 1,0 m²/g bis 1,5 m²/g und einem Aminosäuregehalt von ≥ 2 %.

3. Zubereitung zur Herstellung von Pflege- und kosmetischen Produkten sowie von Nahrungsergänzungsmittel-Produkten,
**gekennzeichnet durch** ein geschmacklich artspezifisches Pulver eines Süßwasserperlen-Hydrolysats, insbesondere eines chinesischen Süßwasserperlen-Hydrolysats, mit einer Korngrößenverteilung von 0,2 µm bis 200 µm mit einer spezifischen Oberfläche von 1,0 m²/g bis 1,5 m²/g und einem Gehalt an organischen Polymeren (Protein, Chitin) von ≥ 0,55 %.

4. Zubereitung zur Herstellung von Pflege- und kosmetischen Produkten sowie von Nahrungsergänzungsmittel-Produkten,
**gekennzeichnet durch** ein geschmacklich artspezifisches Pulver eines Süßwasserperlen-Hydrolysats, insbesondere eines chinesischen Süßwasserperlen-Hydrolysats, mit einer Korngrößenverteilung von 0,5 µm bis 250 µm mit einer spezifischen Oberfläche von 1,0 m²/g bis 1,5 m²/g und einem Aminosäuregehalt von ≥ 2 %.

5. Zubereitung nach Anspruch 1 oder 2,
**dadurch gekennzeichnet,**
**dass** das Süßwasserperlen-Pulver neben organischen Polymeren
| | |
|---|---|
| Calcium (Ca) | > 25 % |
| Blei (Pb) | < 0,5 mg/KG |
| Arsen (As) | < 0,5 mg/KG |
| Quecksilber (Hg) | < 0,1 mg/KG |
| Wasser (H²O) | ≤ 2,0 % |
enthält.

6. Zubereitung nach Anspruch 3 oder 4,
**dadurch gekennzeichnet,**
**dass** das Süßwasserperlen-Pulver-Hydrolysat neben organischen Polymeren
| | |
|---|---|
| Calcium (Ca) | < 20 % |
| Blei (Pb) | < 0,5 mg/KG |
| Arsen (As) | < 0,5 mg/KG |
| Quecksilber (Hg) | < 0,1 mg/KG |
| Wasser (H²O) | ≤ 10 %. |
enthält.

7. Zubereitung nach einem der Ansprüche 1 bis 6,
**dadurch gekennzeichnet,**
**dass** die Zubereitungen folgende Aminosäuren enthalten:
Aspartic Acid, Threonine, Serine, Glutamic, Proline, Glycine, Cysteine, Alanine, Valine, Methione, Isoleucine, Leucine, Tyrosine, Phenylalanine, Lysine, Histidine, Arginine, Ornithine.

8. Zubereitung nach einem der Ansprüche 1 bis 7,
**dadurch gekennzeichnet,**
**dass** die Zubereitung einen synergistischen UV-Schutz umfasst.

9. Zubereitung nach einem der Ansprüche 1 bis 8,
**dadurch gekennzeichnet,**
**dass** die Zubereitung einen synergistischen UV-A-Schutz und/oder einen synergistischen UV-B-Schutz umfasst.

10. Zubereitung nach Anspruch 9,
**dadurch gekennzeichnet,**
**dass** der synergistische UV-B-Schutz in Verbindung mit einer 4-Aminobenzoesäure, Homomenthylsalicylat, 2-Phenylbenzimidazol-5-Sulfonsäure, 4-Dimethylaminobenzoesäure-2-ethylhexylester, 4-Methoxy-zimtsäureisoamylester, 4-Methoxy-zimtsäure-2-ethylhexylester, 3-(4'-Methyl)benzyliden-bornan-2-on, oder anorganischen UV Filtern (Titandioxid bzw. Zinkoxid) und der synergistische UV-A-Schutz in Verbindung mit 2-Hydroxy-4-methoxybenzophenon, 1-(4'-Isopropylphenyl)3-phenyl-1-propan-1,3-dion oder 1-(4-t-Butylphenyl)-3-(4-methoxyphenyl)-propan-1,3-dion oder anorganischen UV-Filtern (Titandioxid bzw. Zinkoxid) erzielt wird.

11. Zubereitung nach einem der Ansprüche 1 bis 10,
hergestellt durch,
- Auswahl von geeigneten Süßwasserperlen
- Waschen und Desinfizieren der Perlen
- Trocknen der Perlen
- Zerkleinern der Süßwasserperlen zu einem Pulver mittels eines Ultra-Mikro-Mahlprozesses
- Saure Hydrolyse für die Herstellung des Süßwasserperlen-Hydrolysats
- Trocknung des Hydrolysats
- Verpackung des Endproduktes.

12. Verwendung eines geschmacksneutralen Pulvers mit einer Korngrößenverteilung von 0,2 µm bis 600 µm mit einer spezifischen Oberfläche von 1,0 m²/g bis 1,5 m²/g und einem Gehalt an organischen Polymeren/Aminosäuren (Protein, Chitin) von ≥ 1,85 % der Süßwasserperle, insbesondere einer chinesischen Süßwasserperle, als Zubereitung für die Herstellung von Pflege- und kosmetischen Produkten sowie für Nahrungsergänzungsmittel-Produkten oder als Zubereitung für die Herstellung von Lichtschutzmitteln.

13. Verwendung einer Zubereitung nach einem der Ansprüche 1 bis 12 als Sonnenschutzmittel in Form von Cremes oder einer anderen geeigneten, auf die menschliche Haut auftragbaren Form.

14. Verwendung eines geschmacklich artspezifischen Pulvers mit einer Korngrößenverteilung von 0,2 µm bis 200 µm mit einer spezifischen Oberfläche von 1,0 m²/g bis 1,5 m²/g und einem Gehalt an organischen Polymeren/Aminosäuren (Protein, Chitin) vom ≥ 0,55 % eines Süßwasserperlen-Hydrolysats, insbesondere eines chinesischen Süßwasserperlen-Hydrolysats, als Zubereitung für die Herstellung von Pflege- und kosmetischen Produkten sowie für Nahrungsergänzungsmittel-Produkten oder als Zubereitung für die Herstellung von Lichtschutzmitteln.

15. Verwendung nach Anspruch 12 oder 14,
**dadurch gekennzeichnet,**
**dass** die Zubereitung bzw. das Lichtschutzmittel mindestens einen UV-Filter umfasst, wobei der UV-Filter ein UV-B-Filter und/oder ein UV-A-Filter ist.

16. Verwendung nach Anspruch 15,
**dadurch gekennzeichnet,**
**dass** die Zubereitung bzw. das Lichtschutzmittel ein Mikropigment umfasst.

17. Verwendung nach Anspruch 16,
**dadurch gekennzeichnet,**
**dass** das Mikropigment ultrafeines Titandioxid ist.
